# FASCICULE DE BREVET EUROPEEN

(11) **EP 3 506 985 B1**
(45) Date de publication et mention de la délivrance du brevet: **29.04.2020**
(21) Numéro de dépôt: 17761476.5
(22) Date de dépôt: 31.08.2017
(51) Int. Cl.: A61Q 19/00, A61K 8/97, A61K 36/45

(54) **UTILISATION COSMETIQUE D'UN EXTRAIT DE FRUIT D'ARBUTUS UNEDO**
KOSMETISCHE VERWENDUNG EINES EXTRAKTES DER ARBUTUS UNEDO
COSMETIC USE OF AN EXTRACT OF ARBUTUS UNEDO

(30) Priorité: 05.09.2016 FR 1670486
(43) Date de publication de la demande: 10.07.2019
(73) Titulaire: Laboratoires Clarins, 75823 Paris Cedex 17 (FR)
(72) Inventeur: COURTIN, Olivier, 92200 Neuilly sur Seine (FR); WEBER, Sandrine, 95830 Cormeilles en Vexin (FR); BLANCHET, Natacha, 78700 Conflans Sainte Honorine (FR)
(74) Mandataire: Gevers & Orès
(86) Numéro de dépôt international: PCT/EP2017/071841
(87) Numéro de publication internationale: WO 2018/041936

(56) Documents cités:
- WO-A1-2013/020182
- FR-A1- 2 736 263
- US-A1- 2014 154 195
- DATABASE GNPD [Online] MINTEL; août 2011 (2011-08), "Orchid+Peach Eye Lifting Cream", XP002769453, Database accession no. 1603696

## Description

La présente invention concerne une composition cosmétique comprenant un extrait de fruit d'Arbutus unedo. La présente invention concerne également l'utilisation cosmétique d'un extrait de fruit d'Arbutus unedo ou d'une composition cosmétique comprenant ledit extrait, en particulier pour réguler les peaux grasses. L'invention çoncerne aussi l'utilisation cosmétique d'un extrait de fruit d'Arbutus unedo ou d'une composition cosmétique comprenant ledit extrait pour inhiber la prolifération des sébocytes ainsi que pour réduire la lipogenèse induite par un stress des sébocytes. L'invention concerne enfin l'utilisation cosmétique d'un extrait de fruit d'Arbutus unedo comme agent matifiant et/ou purifiant, ou dans une composition cosmétique matifiante et/ou purifiante.

La peau est un organe particulier du corps humain. De fine épaisseur, elle est très étendue puisqu'elle recouvre toute la surface du corps et totalise chez l'adulte une surface d'environ 1,6 m². Elle a pour fonction de protéger les tissus profonds du milieu extérieur, tant au niveau de la pénétration physique de corps étrangers, que de l'immunité, de la régulation de la température, de la déperdition de fluides. Enfin, les contraintes mécaniques auxquelles elle est soumise en permanence lui imposent, plus que tout autre organe, une structure solide et cohérente associée à une grande souplesse. Malgré quelques différences d'aspect suivant le site anatomique, elle présente toujours la même structure morphologique de base. Elle est constituée à sa surface de l'épiderme, en profondeur du derme, et plus profondément de l'hypoderme.

La peau est constituée, entre autres, de glandes sébacées situées au niveau du derme et presque toujours associées à un poil. Toutes les parties du corps en sont pourvues, à l'exception de la plante des pieds et de la paume des mains. Certaines zones comme le visage, le cuir chevelu, le torse en comportent un plus grand nombre (environ cinq millions). Ces glandes sébacées sécrètent le sébum qui s'écoule le long du canal pilaire jusqu'à la surface de la peau où, au contact de la sueur, il forme le film hydrolipidique qui hydrate la couche cornée et la protège.

La glande sébacée est une glande acineuse en grappe. Elle est constituée de nombreuses couches cellulaires dans lesquelles on trouve deux types de cellules. D'une part des cellules indifférenciées (couche germinative), situées vers la périphérie de la glande, qui se divisent activement. Ces cellules migrent en 2 semaines environ vers le centre pour donner des cellules différenciées. D'autre part des cellules différenciées centrales (sébocytes) qui contiennent l'équipement enzymatique nécessaire à la synthèse des lipides. Ces cellules ne se divisent plus. En 8 jours elles se transforment en cellules matures plus grosses, remplies de sébum ; les lipides y sont synthétisés et stockés pour constituer finalement de grosses vacuoles.

Dans l'espèce humaine, chaque follicule se développe selon son propre cycle, c'est-à-dire qu'une glande peut s'atrophier pendant qu'une autre s'hypertrophie. Le renouvellement des sébocytes est d'environ 3 semaines.

Le volume glandulaire dépend de l'activité proliférative du compartiment germinatif, du temps nécessaire à la différenciation du sébocyte et de la quantité du sébum synthétisé par chaque sébocyte. Le sébum est produit en quantité plus ou moins importante, en fonction de la taille et du nombre des glandes sébacées. Une sécrétion trop abondante entraîne une modification de la peau : elle devient grasse avec un aspect luisant, son grain est épais et ses pores dilatés avec parfois des points noirs.

Outre les facteurs hormonaux liés à l'adolescence, un certain nombre de facteurs extrinsèques (stress, facteurs climatiques, pollution par exemple) et de facteurs intrinsèques (inflammation, neuropeptides par exemple) peuvent influer sur la sécrétion excessive de sébum.

La demanderesse a mis en évidence de manière surprenante qu'un extrait de fruit d'Arbutus unedo régule les peaux grasses. On peut donc utiliser un extrait de fruit d'Arbutus unedo comme agent régulateur des peaux grasses, pour inhiber la prolifération des sébocytes et/ou pour réduire la lipogenèse induite par un stress des sébocytes.

On entend par agent régulateur des peaux grasses, un agent cosmétique capable de limiter la sécrétion sébacée, de resserrer les pores dilatés, et donc de réduire l'aspect brillant caractéristique des peaux grasses.

L'utilisation selon l'invention est particulièrement adaptée à une application à des peaux saines . Dans le cadre de la présente invention, on désigne par peau saine une peau qui ne présente pas de pathologie cutanée.

Arbutus unedo, communément appelé arbousier en France, est un arbuste de forme buissonnante de la famille des Ericaceae de taille variable généralement entre 1 et 3 mètres. Son fruit, l'arbouse est une baie charnue et globuleuse d'environ 2cm de diamètre et recouverte d'une enveloppe hérissée de petites pointes coniques. Tout d'abord vert, le fruit va maturer durant une année en devenant successivement jaune puis orange pour finalement prendre une teinte écarlate l'hiver suivant. *Arbutus unedo* est présent en Europe méridionale. En France, il est ainsi retrouvé dans le Midi de la France, en Corse, dans les Pyrénées-Orientales, dans le sud-ouest et tout au long du littoral de la Loire-Atlantique.

Consommée en petite quantité, la baie est antidiarrhéique alors qu'en quantité plus importante elle provoque des effets laxatifs. De plus elle possède des propriétés antiinflammatoires, antirhumatismales, diurétiques, digestives et stomachiques.

Les feuilles d'Arbutus unedo sont utilisées comme antiinflammatoire, antiseptique urinaire et également anti diarrhéique. En cosmétique, elles sont très utilisées comme antioxydant, ou dépigmentant du fait de la présence d'arbutine.

Prise en décoction la racine aurait une action antihypertensive, un effet antiinflammatoire et antirhumatismal. Les racines contiennent des glucosides comme par exemple l'arbutoside. Ce dernier est un antiseptique qui aurait joué un grand rôle pour lutter contre les infections urinaires, les maladies sexuellement transmissibles telles que la gonorrhée, ou encore pour traiter l'incontinence.

L'extrait selon l'invention est préférentiellement un extrait de fruit d'Arbutus unedo. De façon avantageuse, cet extrait est un extrait obtenu par extraction au CO₂ supercritique utilisant un co-solvant de triglycérides caprylique/caprique. L'extrait ainsi obtenu est donc contenu dans un co-solvant de triglycérides caprylique/caprique. Il est obtenu selon un procédé comprenant au moins les étapes suivantes :
- Récolte des fruits d'Arbutus unedo qui sont mis à sécher.
- Broyage dans un broyeur réfrigéré.
- Extraction au CO₂ supercritique à l'aide du co-solvant Triglycéride Caprique/Caprylique. L'extraction est effectuée à une pression de 180 bars, et une température de 60°C, le ratio CO₂/quantité de fruit étant égal à 30.
- Séchage sous vide de l'extrait (à pression de 20 mbars et température de 55 à 65°C).

L'extrait de fruit d'Arbutus unedo utilisé selon l'invention est un liquide limpide orangé d'odeur caractéristique. Il présente les caractéristiques analytiques suivantes :
- Densité relative à 20°C : 0.94-.96
- Indice de réfraction à 20°C : 1.440-1.452
- Indice d'acide (mg KOH/g) : ≤ 10
- Indice de saponification (mg KOH/g) : 325-360
- Indice de peroxyde (meq O₂/kg) : ≤ 10
- Indice d'iode (g I₂/100g) : ≤ 10
- Insaponifiables : ≥ 0.8 %
- Contenu en eau ≤ 0.2%

De préférence la composition cosmétique selon l'invention comprend de 0,01 à 10% d'un extrait de fruit d'Arbutus unedo en poids de matière sèche par rapport au poids total de la composition. Avantageusement, la composition comprend de 0,01 à 5% d'un extrait de fruit d'Arbutus unedo en poids de matière sèche par rapport au poids total de la composition.

Les compositions selon l'invention peuvent encore comprendre un ou plusieurs agents de formulation ou additifs d'usage connu et classique dans les compositions cosmétiques et dermatologiques tels que, à titre d'exemples et de façon non limitative, des adoucissants, des colorants, des actifs filmogènes, des tensioactifs, des parfums, des conservateurs, des émulsionnants, des huiles, des glycols, des vitamines comme la vitamine E, des filtres UV, etc... Grâce à ses connaissances en matière de cosmétique, l'homme du métier saura quels agents de formulation ajouter aux compositions de l'invention et en quelles quantités en fonction des propriétés recherchées.

Les compositions selon l'invention peuvent se présenter sous toute forme connue de l'homme du métier dans le domaine de la cosmétologie et de la dermatologie sans autre restriction galénique que l'application sur le visage et sur le corps. De façon avantageuse, les compositions selon l'invention se présentent sous la forme d'un gel, d'une crème, d'une lotion, d'un masque, d'une huile, d'un lait, d'un spray, etc...

La demanderesse a démontré que l'extrait de fruit d'Arbutus unedo utilisé selon l'invention ainsi que la composition cosmétique le comprenant peuvent être utilisés à des fins cosmétiques par application sur la peau pour réguler les peaux grasses, pour inhiber la prolifération des sébocytes ainsi que pour réduire la lipogenèse induite par un stress des sébocytes.

L'extrait de fruit d'Arbutus unedo peut donc être utilisé à des fins cosmétiques comme agent régulateur des peaux grasses, pour inhiber la prolifération des sébocytes ainsi que pour réduire la lipogenèse induite par un stress des sébocytes. Il peut également être utilisé à des fins cosmétiques comme agent matifiant et/ou purifiant.

Par agent matifiant et/ou purifiant, on entend un agent cosmétique capable de donner un aspect plus mat à une peau grasse. Il permet de réduire l'aspect brillant caractéristique des peaux grasses.

L'extrait de fruit d'Arbutus unedo peut aussi être utilisé à des fins cosmétiques comme agent unifiant le grain de la peau et/ou réduisant la taille des pores.

L'extrait de fruit d'Arbutus unedo peut également être utilisé dans une composition cosmétique régulatrice des peaux grasses ou une composition matifiante et/ou purifiante.

L'extrait de fruit d'Arbutus unedo selon l'invention et la composition cosmétique le comprenant sont utilisés par application sur la peau ; il peut s'agir indifféremment de la peau du visage ou du corps.

Lorsque qu'il s'agit de la peau du visage, c'est préférentiellement la peau de la face, et plus particulièrement la zone T du visage c'est à dire la zone front-nez-menton du visage.

Les exemples ci-après concernent, d'une part l'évaluation de l'effet de l'extrait de fruit d'Arbutus unedo sur la prolifération des sébocytes ainsi que sur la différentiation des sébocytes soumis à un stress ; et d'autre part des compositions objets de la présente invention.

Les exemples font référence aux figures suivantes dans lesquelles :
- La figure 1 représente le pourcentage de prolifération des sébocytes par rapport au contrôle pour différentes conditions : le contrôle, la cytosine-b-D-arabinofuranoside et l'extrait d'Arbutus unedo à 0.001%, 0.005% et 0.01%.
- La figure 2 représente l'intensité des gouttelettes lipidiques en fonction des différentes concentrations d'extrait d'Arbutus unedo à 0.001%, 0.005% et 0.01% ainsi que le témoin non traité et le témoin positif traité seulement à la CRH (Corticotropin Releasing Hormone).

### I. EVALUATION DE L'ACTIVITE D'UN EXTRAIT D'ARBUTUS UNEDO SUR LA PROLIFERATION DES SEBOCYTES EN CULTURE

### A.MATERIEL ET METHODE

Les sébocytes sont ensemencés dans une plaque 96 puits blanche à raison de 12 500 cellules par puits dans du milieu SEB1 (ZenBio). Après 24 heures, les cellules sont traitées avec l'extrait d'Arbutus unedo à 0.001%, 0.005% et 0.01% ainsi qu'avec la référence (cytosine-b-D-arabinofuranoside) à 1*µ*mol/L.

Après 24 heures d'incubation, une solution de 5-bromo-2' deoxyuridine (BrdU) diluée à 10*µ*M final est ajoutée aux solutions de traitement. L'incorporation du BrdU est mesurée 24 heures plus tard, après fixation des cellules puis ajout d'un anticorps anti-BrdU.

Une solution de révélation est ensuite ajoutée et la prolifération des sébocytes est calculée par mesure de la luminescence.

### B.RESULTATS

La cytosine-b-D-arabinofuranoside, contrôle positif, inhibe totalement la prolifération des sébocytes en culture (-94.8%).

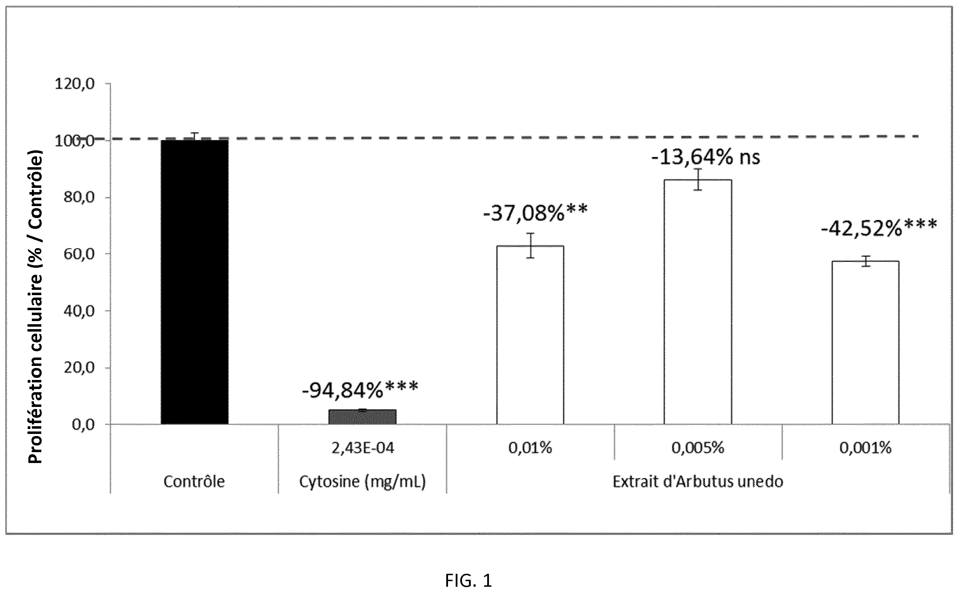

Dans ces conditions expérimentales, les résultats (figure 1) permettent de montrer que l'extrait de fruit d'Arbutus unedo a une activité inhibitrice sur la prolifération des sébocytes aux doses de 0.01% (-37%) et 0.001% (-42,5%).

### II. EVALUATION DE L'ACTIVITE D'UN EXTRAIT D'ARBUTUS UNEDO SUR LA DIFFERENTIATION DES SEBOCYTES EN CULTURE SOUMIS A UN STRESS

### A.MATERIEL ET METHODE

Les sébocytes sont ensemencés dans des chambres de culture 8 puits à raison de 12000 cellules par puits dans du milieu SEB1 (ZenBio) . Le milieu de culture est changé tous les 2/3 jours.

A J8 après l'ensemencement, les cellules sont prétraitées avec l'extrait de fruit d'Arbutus unedo à 0.001%, 0.005% et 0.01%

Après 24h, la différenciation des sébocytes est induite par stress chimique en ajoutant au milieu de culture la CRH (Corticotropin Releasing Hormone) (10⁻⁷M) pendant 3 jours. Cette hormone est l'hormone du stress. Elle a pour fonction de moduler l'axe hypophyso-corticosurrénalien en réponse au stress. La corticotrophine va se fixer sur des récepteurs à la surface des sébocytes. Une fois activé par la corticotrophine, le récepteur produit des cytokines pro-inflammatoires et module la prolifération et la différenciation des sébocytes entrainant l'augmentation de la sécrétion sébacée.

Les chambres de culture sont ensuite fixées à la formaline (15min à température ambiante) puis marquées au Bodipy/Dapi selon le protocole suivant : Triton 10min, BSA 2% 10min, Bodipy/Dapi 1h à température ambiante.

Des clichés sont pris à l'aide du microscope à fluorescence (Nikon 50i, logiciel d'acquisition NIS Elements Nikon) puis sont analysés après post-traitement d'images à l'aide du logiciel Visilog 2.0. (FEI) La quantité de fluorescence mesurée (Relative Fluorescence Units) est proportionnelle à la quantité de lipides intracellulaires accumulés. Toutes les conditions testées sont réalisées en duplicata.

### B.RESULTATS

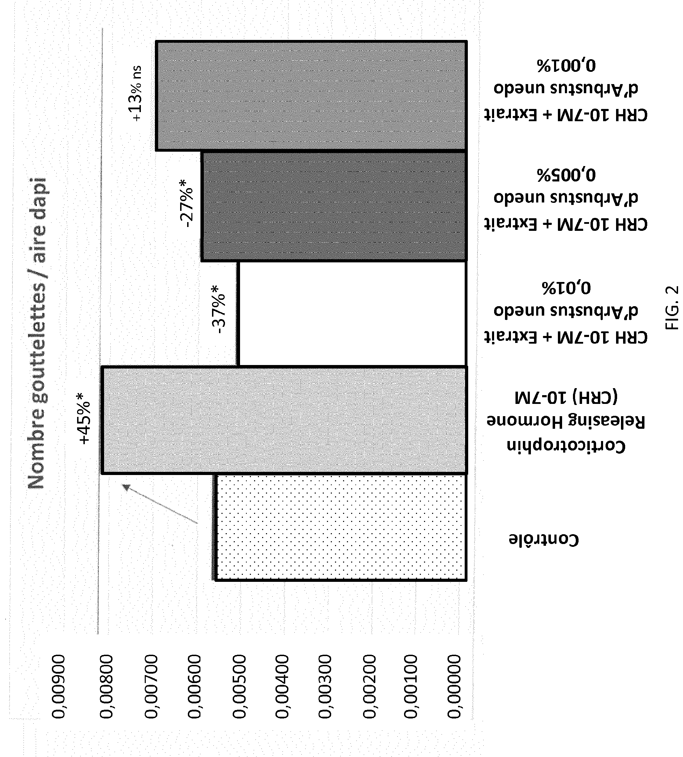

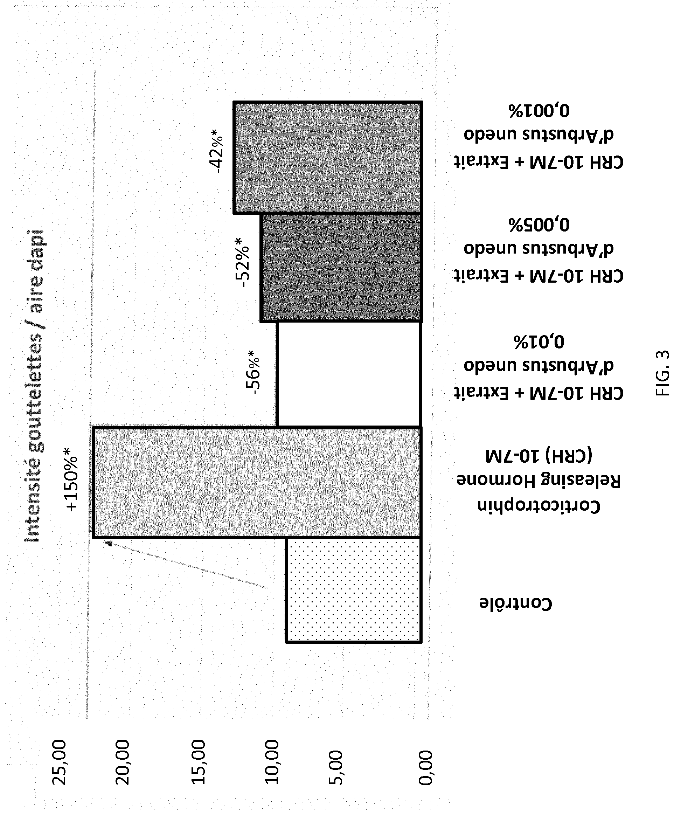

La CRH augmente de 150% l'intensité de fluorescence relative à la quantité de lipides intracellulaires par rapport au témoin non traité. L'extrait de fruit d'Arbutus unedo réduit significativement la lipogenèse induite par un stress des sébocytes en culture par rapport au témoin CRH (figure 2). A la concentration de 0,01%, l'extrait réduit l'intensité des gouttelettes lipidiques de 56%.

### III.EXEMPLES DE COMPOSITIONS

**CREME PEAUX GRASSES**

| | % |
|---|---|
| TETRASODIUM EDTA..... | 0,05 |
| GLYCERINE..... | 4,00 |
| ARISTOFLEX AVC..... | 1,00 |
| SEPIGEL 305..... | 0,60 |
| PARLEAM 4..... | 3,00 |
| CETEARYL ISONONANOATE..... | 7,00 |
| SILICONE DC 1503..... | 1,00 |
| GLUCONATE DE ZINC..... | 0,02 |
| CHLORYDRATE DE PYRIDOXINE..... | 0,10 |
| ALCOOL DENATURE..... | 5,00 |
| ACIDE SALICYLIQUE..... | 0,20 |
| NaOH..... | 0,07 |
| GLYCOFILM..... | 3,00 |
| SPHERICA P1500..... | 3,00 |
| EXTRAIT D'ARBUTUS UNEDO..... | 0,10 |
| CONSERVATEURS..... | 1,20 |
| PARFUM..... | 0,30 |
| EAU DEMINERALISEE..... | Q.S.P 100 |

**SERUM PEAUX GRASSES**

| | % |
|---|---|
| DISODIUM EDTA..... | 0,10 |
| PENTYLENE GLYCOL..... | 1,00 |
| GOMME XANTHANE..... | 0,20 |
| COVACRYL MV60..... | 0,40 |
| GLYCERINE..... | 2,00 |
| BUTYLENE GLYCOL..... | 2,00 |
| AMPHISOL K..... | 0,35 |
| MONTANOV L..... | 2,00 |
| ALCOOL CETYLIQUE..... | 0,50 |
| MONOSTEARATE DE GLYCEROL..... | 1,50 |
| ISONONYL ISONONANOATE..... | 3,00 |
| DIMETHICONE..... | 1,50 |
| CETEARYL OCTANOATE..... | 5,00 |
| TOCOPHEROL..... | 0,02 |
| SODIUM HYALURONATE..... | 0,20 |
| METHYL METHACRYLATE CROSSPOLYMER..... | 0,50 |
| HEXYL RESORCINOL & GLYCERINE..... | 2,50 |
| SEPIGEL 305..... | 0,50 |
| EXTRAIT D'ARBUTUS UNEDO..... | 0,10 |
| CONSERVATEURS..... | 0,56 |
| PARFUM..... | 0,30 |
| EAU DEMINERALISEE..... | Q.S.P 100 |

**LOTION PEAUX GRASSES**

| | % |
|---|---|
| DISODIUM EDTA..... | 0,20 |
| SODIUM CITRATE..... | 0,20 |
| CHLORURE DE SODIUM..... | 0,75 |
| PROPYLENE GLYCOL..... | 1,00 |
| PENTYLENE GLYCOL..... | 2,00 |
| D-PANTHENOL..... | 0,25 |
| CYTOBIOL IRIS..... | 0,50 |
| ALOE VERA..... | 2,00 |
| EXTRAIT D'ARBUTUS UNEDO..... | 0,10 |
| CONSERVATEURS..... | 0,56 |
| PARFUM..... | 0,20 |
| EAU DEMINERALISEE..... | Q.S.P 100 |

## Revendications

1. Utilisation cosmétique d'un extrait de fruit d'Arbutus unedo comme agent régulateur des peaux grasses.

2. Utilisation cosmétique selon la revendication 1, dans laquelle l'extrait de fruit d'Arbutus unedo est un agent matifiant et/ou purifiant.

3. Utilisation cosmétique selon la revendication 1, pour inhiber la prolifération des sébocytes.

4. Utilisation cosmétique selon la revendication 1, pour réduire la lipogenèse induite par un stress des sébocytes.

5. Utilisation cosmétique selon la revendication 1, dans laquelle l'extrait de fruit d'Arbutus unedo est un agent unifiant le grain de la peau et/ou réduisant la taille des pores.

6. Utilisation cosmétique d'un extrait de fruit d'Arbutus unedo selon l'une quelconque des revendications 1 à 5, dans une composition cosmétique régulatrice des peaux grasses.

7. Utilisation cosmétique d'un extrait de fruit d'Arbutus unedo selon l'une quelconque des revendications 1 à 5, dans une composition cosmétique matifiante et/ou purifiante.

8. Utilisation selon l'une quelconque des revendications 1 à 7 dans laquelle l'extrait de fruit d'Arbutus unedo est un extrait obtenu par extraction au CO₂ supercritique.

9. Utilisation selon l'une quelconque des revendications 1 à 7 dans laquelle l'extrait de fruit d'Arbutus unedo est un extrait contenu dans un co-solvant de triglycérides caprylique/caprique.

## Patentansprüche

1. Kosmetische Verwendung eines Fruchtextrakts des Arbutus unedo als Regulierungsmittel fettiger Haut.

2. Kosmetische Verwendung nach Anspruch 1, wobei der Fruchtextrakt des Arbutus unedo ein mattierendes und/oder reinigendes Mittel ist.

3. Kosmetische Verwendung nach Anspruch 1, um die Verbreitung der Sebozyten zu hemmen.

4. Kosmetische Verwendung nach Anspruch 1, um die durch eine Belastung der Sebozyten induzierte Lipogenese zu verringern.

5. Kosmetische Verwendung nach Anspruch 1, wobei der Fruchtextrakt des Arbutus unedo ein Mittel ist, das die Hautstruktur vereinheitlicht und/oder die Porengröße verringert.

6. Kosmetische Verwendung eines Fruchtextrakts des Arbutus unedo nach einem der Ansprüche 1 bis 5, in einer fettige Haut regulierenden kosmetischen Zusammensetzung.

7. Kosmetische Verwendung eines Fruchtextrakts des Arbutus unedo nach einem der Ansprüche 1 bis 5, in einer mattierenden und/oder reinigenden kosmetischen Zusammensetzung.

8. Verwendung nach einem der Ansprüche 1 bis 7, wobei der Fruchtextrakt des Arbutus unedo ein Extrakt ist, der durch überkritische CO₂-Extraktion erhalten wird.

9. Verwendung nach einem der Ansprüche 1 bis 7, wobei der Fruchtextrakt des Arbutus unedo ein Extrakt ist, der in einem Cosolvens aus Capryl-/Caprinsäure Triglycerid enthalten ist.

## Claims

1. Cosmetic use of an extract of Arbutus unedo fruit as an oily skin regulating agent.

2. Cosmetic use according to claim 1, wherein the extract of the Arbutus unedo fruit is a matting and/or purifying agent.

3. Cosmetic use according to claim 1, to inhibit the proliferation of sebocytes.

4. Cosmetic use according to claim 1, to reduce the lipogenesis induced by a stress of the sebocytes.

5. Cosmetic use according to claim 1, wherein the extract of the Arbutus unedo fruit is an agent that unifies the texture of the skin and/or reduces the size of the pores.

6. Cosmetic use of an extract of Arbutus unedo fruit according to any one of claims 1 to 5, in a cosmetic oily skin regulating composition.

7. Cosmetic use of an extract of Arbutus unedo fruit according to any one of claims 1 to 5, in a matting and/or purifying cosmetic composition.

8. Use according to any one of claims 1 to 7, wherein the extract of the Arbutus unedo fruit is an extract obtained by extraction with supercritical CO₂.

9. Use according to any one of claims 1 to 7, wherein the extract of the Arbutus unedo fruit is an extract contained in a caprylic/capric triglyceride co-solvent.
